# EUROPEAN PATENT SPECIFICATION

(11) **EP 2 273 999 B1**
(45) Date of publication and mention of the grant of the patent: **22.03.2017**
(21) Application number: 09729322.9
(22) Date of filing: 07.04.2009
(51) Int. Cl.: A61K 36/258

(54) **COMPOSITION COMPRISING POLYSACCHARIDE EXTRACTED FROM PANAX GINSENG PREVENTING AND TREATING LIVER DISEASES**
ZUSAMMENSETZUNG MIT AUS PANAX GINSENG EXTRAHIERTEM POLYSACCHARID ZUR PRÄVENTION UND BEHANDLUNG VON LEBERERKRANKUNGEN
COMPOSITION COMPRENANT UN POLYSACCHARIDE EXTRAIT DE PANAX GINSENG DESTINÉE À LA PRÉVENTION ET AU TRAITEMENT DE MALADIES HÉPATIQUES

(30) Priority: 07.04.2008 KR 20080032386
(43) Date of publication of application: 19.01.2011
(73) Proprietor: Korea Institute of Radiological & Medical Sciences, Nowon-gu, Seoul 139-240 (KR)
(72) Inventor: YUN, Yeon-Sook, Seoul 135-280 (KR); SONG, Jie-Young, Goyang-si Gyeonggi-do 410-716 (KR); JUNG, In-Sung, Seoul 110-530 (KR)
(74) Representative: Leonard, Thomas Charles
(86) International application number: PCT/KR2009/001789
(87) International publication number: WO 2009/125964

(56) References cited:
- WO-A1-95/02968
- KR-B1- 100 190 756
- US-A1- 2003 190 378
- SONG JLE-YOUNG ET AL: "Effects of polysaccharide ginsan from Panax ginseng on liver function", ARCHIVES OF PHARMACAL RESEARCH, NATL. FISHERIES UNIVERSITY, PUSAN, KR, vol. 27, no. 5, 1 May 2004 (2004-05-01), pages 531-538, XP008095595, ISSN: 0253-6269, DOI: 10.1007/BF02980127
- LEE Y-S ET AL: "ACTIVATION OF MULTIPLE EFFECTOR PATHWAYS OF IMMUNE SYSTEM BY THE ANTINEOPLASTIC IMMUNOSTIMULATOR ACIDIC POLYSACCHARIDE GINSAN ISOLATED FROM PANAX GINSENG", ANTICANCER RESEARCH, INTERNATIONAL INSTITUTE OF ANTICANCER RESEARCH, GR, vol. 17, no. 1A, 1 January 1997 (1997-01-01), pages 323-331, XP001117999, ISSN: 0250-7005
- YEON SOOK YUN1 ET AL: "Inhibition of Autochthonous Tumor by Ethanol Insoluble Fraction from Panax ginseng as an Immunomodulator", PLANTA MEDICA, THIEME VERLAG, DE, vol. 59, no. 6, 1 December 1993 (1993-12-01), pages 521-524, XP009168746, ISSN: 0032-0943, DOI: 10.1055/S-2006-959752
- ASSINEWE V A ET AL: "Extractable polysaccharides of Panax quinquefolius L. (North American ginseng) root stimulate TNFalpha production by alveolar macrophages", MEDICINAL & AROMATIC PLANTS ABSTRACTS, SCIENTIFIC PUBLISHERS, SCIENTIFIC PUBLISHERS, NEW DELHI - INDIA, vol. 25, no. 4, 1 August 2003 (2003-08-01) , XP018012694, ISSN: 0250-4367
- LIM, T-S. ET AL: "Immunomodulating activities of polysaccharides isolted from Panax gingseng", JOURNAL OF MEDICAL FOOD, vol. 7, no. 1, 7 July 2004 (2004-07-07), pages 1-6, XP002698657,
- Jie-Young Song ET AL: "Radioprotective effects of ginsan, an immunomodulator.", Radiat Res, 1 January 2003 (2003-01-01), pages 768-774, XP55059649, Retrieved from the Internet: URL:http://www.ginsan.com.hk/documents/gin san12.pdf [retrieved on 2013-04-15]
- BATALLER R ET AL: "Liver fibrosis", JOURNAL OF CLINICAL INVESTIGATION, AMERICAN SOCIETY FOR CLINICAL INVESTIGATION, US, vol. 115, no. 2, 1 February 2005 (2005-02-01), pages 209-218, XP002450194, ISSN: 0021-9738, DOI: 10.1172/JCI200524282
- ASSINEWE V. A. ET AL.: 'Extractable polysaccharides of Panax quinquefolius L. (North American ginseng) root stimulate TNFa production by alveolar macrophages' PHYTOMEDICINE vol. 9, no. 5, 2002, pages 398 - 404, XP004957107

## Description

### Technical Field

The present invention relates to a white Panax ginseng polysaccharide extract, which is useful for the prevention and treatment of liver diseases, and a method for preparing the same. Further, the present invention relates to a composition for the prevention and treatment of liver diseases, comprising the white Panax ginseng polysaccharide extract as an active ingredient.

### Background Art

In accordance with the annual report for 2001 on the cause of death statistics, published by Korea National Statistical Office, liver disease was the fifth leading cause of death in Korea, and Korea (22.3 persons) ranked third among 29 OECD countries providing cause of death data on [OECD Health Data], after Hungary (57.3 persons) and Mexico (43.2 persons). To date, there are approximately 6∼8 hundred million patients worldwide who have chronic liver disease. However, since the liver is a silent organ, liver disease has no obvious symptoms until progressed to severe liver injury. For that reason, it is difficult to recognize liver disease during its early stages, and only about half of the patients can benefit from currently available therapeutic agents. Up to now, there have been no FDA-approved therapeutic agents for liver injury or prophylactic agents for cirrhosis. Therefore, many studies have been made to develop therapeutic agents for liver disease using natural substances that are not harmful to the human body with less side effects, rather than organic chemicals, so that long term use of the therapeutic agent does not cause adverse effects on the liver, and it can be also easily taken by the patients.

The liver is, more than any other organ, actively involved in the metabolism producing thousands of enzymes essential to the human body. The liver is located between the digestive system and the circulatory system, and critical in protecting the body from a variety of harmful substances. Since foreign substances should pass through the liver, it is exposed to harmful substances as well as nutrients, and thus liver is at much higher risk of damage than other organs. However, the liver is usually one of the body's most resilient organs and is capable of regenerating after damage or injury. When the liver is exposed to continuous stimulations such as chronic fatigue, excessive intake of high fat foods or alcohol, harmful substances including various chemicals, nutritional deficiency, virus infection (HCV, HBV), and autoimmune diseases, liver injury occurs, resulting from hepatic inflammation and liver cell death. In particular, excessive intake of high fat foods or alcohol causes fatty liver, which is stroma accumulation in the liver tissue, and increases the levels of serum sGOT (serum glutamate-oxaloacetate transaminase) and serum sGPT (serum glutamate-pyruvate transaminase). After liver damage, hepatic stellate cells become activated, leading to hepatic fibrosis via hepatitis. Subsequently, hepatic fibrosis progresses to liver cirrhosis, following liver cancer. Liver cirrhosis is commonly considered to be an irreversible state, resulting from repeated process of hepatic fibrosis, and is accompanied by severe complications such as liver cancer, ascites, peritonitis, and variceal bleeding.

Recently, animal models greatly contribute to the development of therapeutic agents for liver disease, and carbon tetrachloride (CCL₄)-induced animal models are used to develop therapeutic agents for liver disease caused by toxins. Carbon tetrachloride, causing fatty degeneration, fibrosis, hepatocellular death and carcinogenesis, is a representative toxin that is generally used to investigate the effects of toxins on liver metabolism, and its hepatotoxicity has been widely studied employing the methods such as incubation of hepatic cells or liver slices with carbon tetrachloride and direct injection to mouse or rat. Carbon tetrachloride is metabolized by cytochrome P450 that plays a multifunctional role in the liver, and converted into highly toxic free radicals, CCl₃ and Cl. Free radical CCl₃ causes oxidation of triglycerides accumulated in the liver or fatty acids in membrane phospholipids, leading to lipid oxidation, and binds to oxygen to facilitate oxidation with lipids. Such lipid peroxidation accumulates lipids in the liver, and reduces protein synthesis, leading to glycogen decomposition, and affects cellular structure such as mitochondria, DNA, and cell membrane, resulting in cell damage and death (Recknagel, R. O., et al., Pharmacol.Rev., 19, pp145-208, 1967; Chang, LM., et al., Drug and chemical toxicology, 6(5), pp442-453, 1983). In addition, lipid peroxidation activates TNF-α(tumor necrosis factor), NO (Nitric oxide), TGF-β(transforming growth factor) or the like, so as to induce cell necrosis or fibrosis (Sato et al., The protective effect of hepatocyte growth-promoting factor (pHGF) against carbon tetrachloride-induced acute liver injury in rat: an ultrastructural study, Med Eletron Microsc 32, 184-192. 1999). That is, carbon tetrachloride inhibits protein biosynthesis in the liver, and causes the elevation of serum GOT and GPT levels and centrilobular hepatocyte necrosis histologically.

General therapies for liver diseases are largely divided into diet and drug therapies, and the combination of these two therapies is actually employed in the treatment of liver diseases. Currently, silymarin and biphenyl dimethyl dicarboxylate (BDD) are used as the therapeutic agent for liver diseases. Actually, since liver diseases are not caused by one factor but resulted from the complex action of various factors, there are no drugs regarded as the ideal remedy, yet. Accordingly, satisfactory therapeutic effects on liver diseases cannot be attained by drugs with one action mechanism. For example, rest and high protein diet plus vitamins are recommended for the treatment of acute liver disease, in stead of drug therapy. Drugs such as silymarin and BDD are used for a therapeutic purpose, but not a prophylactic purpose, namely, they are only required for the treatment of hepatitis.

Animal models have been used to develop a number of drugs capable of protecting liver function and preventing or treating hepatitis, for instance, *Cichorium Intybus* has been used for liver diseases for an age in India, in which an active ingredient, cichorin of 75 mg/kg was found to show an effect of protecting liver function in mouse exposed to carbon tetrachloride (H.Kalantari Ph et al., Protective Property of Cichorium Intybus in CCl4 induced Liver Damage in mice. School of Pharmacy, Ahwaz university of Medical Sciences, Ahwaz, Iran.). Glycyrrhizin extracted from the root of liquorice, chemically a triterpenoid saponin, is GRAS (Generally Recognized as Safe) classified in US, and thus used in candy, medicine, and cigarette. Also, it was reported that glycyrrhizin has inhibitory effects on cancer and tumor and hepatoprotective effect, and also improves arteriosclerosis, but its excessive use may cause sexual dysfunction, hypertension and edema. In addition to the above substances, Curcuma longa L has been reported to possess liver protective activity and anticancer actions, and saponin, bupleuroside compounds (H. Maysuda et al., Bioorg. Med. Chem., 7, pp2193-2198, 1997), naringin (K.Kawaguchi et al., Eur. J. Pharmacol., 368, pp1560-1567, 2001), and polysaccharide extracted from the seed of Celosia argentea have been reported to have hepatoprotective effects and prevent the death of experimental animals in DGalN/ LPS-induced liver injury model (K.Hase et al., Biol. Pharm.. Bull., 19, pp567-572, 1996). Further, it was suggested that plants contain polysaccharides or glycoproteins having hepatoprotective effects or therapeutic effects on hepatitis. Experiments have shown that the polysaccharides or glycoproteins contained in plants significantly reduce hepatotoxicity, and glucan from Saccharomyces cerevisiae stimulates macrophages to enhance resistance against virus (see: Science 208:67-69, 1980). However, there are still no reports that polysaccharides from the plants belonging to the genus *Panax* ginseng exert preventive and therapeutic effects on liver diseases.

Meanwhile, the present inventors have previously reported that Panax ginseng polysaccharide consisting of glucose, galactose, galacturonic acid, and protein has radioprotective effects (Korean Patent No. 144130), and Panax ginseng polysaccharide has hematopoietic, myeloprotecting, and radiosensitizing activities (Korean Patent No. Furthermore, SONG JLE-YOUNG ET AL: "Effects of polysaccharide ginsan from Panax ginseng on liver function",ARCHIVES OF PHARMACAL RESEARCH, NATL. FISHERIES UNIVERSITY, PUSAN, KR, vol. 27, no. 5, 1 May 2004 (2004-05-01), pages 531-538 discloses the effects of a polysaccharide extract, which is isolated from Panax ginseng C.A. Meyer, on the liver functions. However, the polysaccharides reported in the prior arts are different from polysaccharide extracted by the present invention in terms of content and composition, and their action mechanisms on the diseases are also different from each other. Further, the separation and purification methods, described in the above patents, are not advantageous in the development of therapeutic agents, because of complicated, low-yield processes. Furthermore, the present inventors have recognized that change of glucose and galactose contents in the composition greatly affects the activities.

### Disclosure of Invention

### Technical Problem

There is still a need to develop more effective and improved agents for treating hepatitis or protecting liver function, which is also the object of the present invention. Therefore, the present inventors obtained polysaccharide by extraction of white ginseng with water, concentration of the liquid extract, and precipitation with ethanol. They found that the polysaccharide comprises mannose of 1.2∼2.8% by weight, glucose of 64.1∼82.8% by weight, galactose of 10.0∼26.6% by weight, and arabinose of 0.6∼5.4% by weight, and exerts very excellent hepatoprotective and therapeutic effects through various action mechanisms in vitro experiment and carbon tetra-chloride-induced liver injury models, thereby completing the present invention.

The subject matter for which protection is sought is as defined in the claims.

### Technical Solution

Accordingly, it is an object of the present invention to provide a white Panax ginseng polysaccharide extract for use in the prevention and treatment of certain liver diseases.

It is another object of the present invention to provide a method for preparing the white Panax ginseng polysaccharide extract.

It is still another object of the present invention to provide a composition for use in the prevention and treatment of liver diseases, comprising the white Panax ginseng

polysaccharide extract as an active ingredient. It is still another object of the present invention to provide a composition for use in inhibiting hepatic fibrosis, comprising the white Panax ginseng polysaccharide extract as an active ingredient.

### Advantageous Effects

As mentioned above, the white Panax ginseng polysaccharide used in the present

invention significantly reduces liver injury that is induced by hepatotoxins such as carbon tetrachloride and acetaminophen, and effectively modulates inflammation in the body. Accordingly, the white Panax ginseng polysaccharide used in present invention reduces stress- or alcohol-induced liver injury, or drug- or food-induced hepatotoxicity, and also modulates inflammation in the body. In addition, the white Panax ginseng polysaccharide used in the present invention exerts its effects via oral and intraperitoneal administrations, and does not cause adverse effects on the liver upon long-term administration.

### Brief Description of Drawings

FIG. 1 is a graph showing no hepatotoxic action of Panax ginseng polysaccharide after administration by intraperitoneal injection at various concentrations;
FIG. 2 is a graph showing that carbon tetrachloride-induced hepatotoxicity was significantly reduced by the administration of Panax ginseng polysaccharide;
FIG. 3 is a graph showing that acetaminophen-induced liver injury was effectively inhibited by the administration of Panax ginseng polysaccharide;
FIG. 4 is a photograph of a histochemical staining of liver tissue, showing that carbon tetrachloride-induced liver injury was reduced by the administration of Panax ginseng polysaccharide;
*FIG. 5 is a photograph showing that cytochrome P450-2E1 expression was reduced by the administration of Panax ginseng polysaccharide in the carbon tetrachloride-induced liver injury tissue;
FIG. 6 is a photograph showing that the increased chemokine levels were reduced by the administration of Panax ginseng polysaccharide in the carbon tetrachloride-induced liver injury;
FIG. 7 is a photograph showing that the levels of inflammatory cytokines were reduced by the administration of Panax ginseng polysaccharide; and
FIG. 8 is a photograph showing that hepatic fibrosis was inhibited by the administration of Panax ginseng polysaccharide.

### Best Mode for Carrying out the Invention

According to an embodiment to achieve the above objects of the present invention, the present invention relates to a polysaccharide extracted from white Panax ginseng for use in the prevention and treatment of liver diseases.

Examples of the plants belonging to the *Panax* genus include Panax ginseng C.A. Mayer, Panax quinquefolium, Panax notoginseng, Panax pseudoginseng, Panax japonicum, and Panax vietnamensis Ha et Grushv. Ginseng Radix Alba derived from Panax ginseng C.A. Mayer, also called white ginseng, is used in the present invention. The white ginseng is prepared by removing rootlets and stem barks from Panax ginseng C.A. Mayer and drying it.

The polysaccharide according to the present invention comprises sugars including mannose of 1.2∼2.8% by weight, glucose of 64.1∼82.8% by weight, galactose of 10.0∼26.6% by [32] weight, and arabinose of 0.6∼5.4% by weight.

The polysaccharide extracted from white Panax ginseng of the present invention can be used for the prevention and treatment of liver diseases, in particular fatty liver, liver necrosis, hepatic fibrosis, liver cirrhosis and acute/chronic hepatitis.

The present inventors examined the effects of the white Panax ginseng polysaccharide used in the present invention on liver protection by the following methods.

Specifically, the effects of improving liver function were evaluated by using various biomarkers, and the representative biomarkers used in the present invention are serum biomarkers of hepatic injury, such as ALT (Alanine aminotransferase), AST (Aspartate aminotransferase), and ALP (Alkaline phosphatase) enzymes, total bilirubin, and albumin. Their levels are not within the normal range, when hepatocellular necrosis or injury occurs. If a drug exerts protective effects on hepatic cells, the levels return to the normal range.

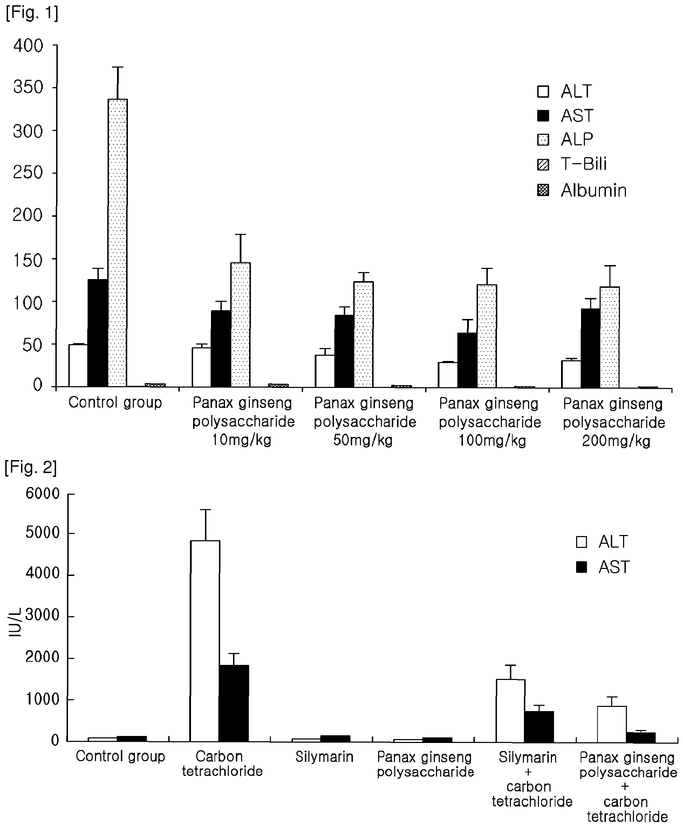

First, the present inventors found that there were no significant differences in the levels of hepatic injury biomarkers between a control group and single administration or long-term administration of Panax ginseng polysaccharide used in the present invention, indicating that the Panax ginseng polysaccharide used in the present invention showed hepatoprotective action without hepatotoxicity (see FIG. 1 and Table 2).

Further, when liver injury was induced by hepatotoxins such as carbon tetrachloride and acetaminophen in mice, the Panax ginseng polysaccharide used in the present invention significantly reduced the levels of serum biomarkers of hepatic injury, generation of lipid peroxide, cytochrome P450-2E1 that is responsible for carbon tetrachloride metabolism, and production of inflammatory cytokines and chemokines that exacerbate liver injury (see FIGs. 2 to 8).

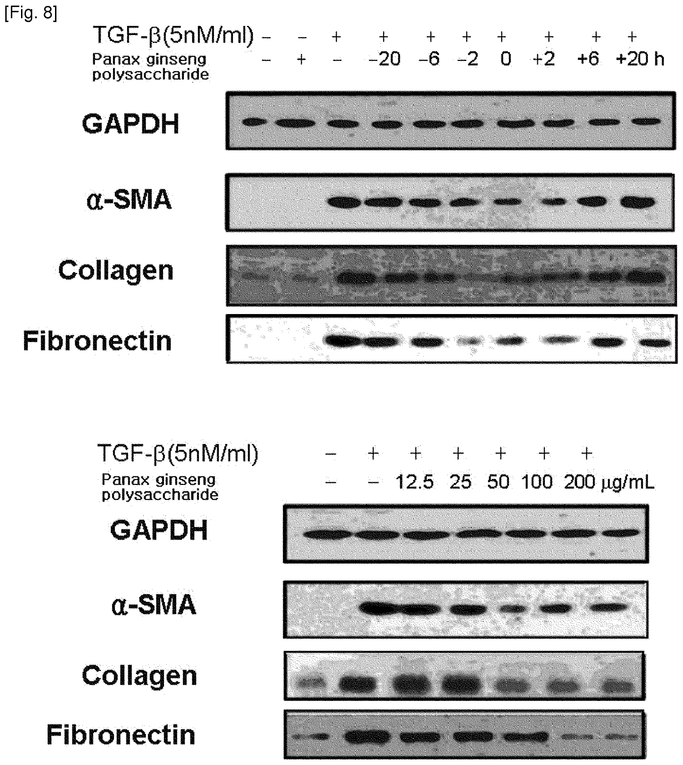

Furthermore, hepatic fibrosis resulting from severe inflammation or tissue transformation was significantly reduced by pre-treatment or post-treatment of the Panax ginseng polysaccharide, indicating its protective and therapeutic effects on hepatic fibrosis (see FIG. 8).

Taken together, it can be seen that the Panax ginseng polysaccharide used in the present invention can be very useful for the prevention and treatment of liver diseases.

In another embodiment, the present invention relates to a method for extracting the white Panax ginseng polysaccharide from a plant of the genus *Panaxginseng.*

The method for extracting the white Panax ginseng polysaccharide according to the present invention comprises the steps of (a) pulverizing and drying the plant of the genus *Panaxginseng,* and then cold-infusing at 0-25°C or heating it at 50-180°C for 0.5-20 hrs to obtain a liquid extract; (b) concentrating the liquid extract by distillation under reduced pressure, and then precipitating and separating the concentrated liquid extract using an organic solvent comprising 70-90% (v/v) ethanol; and (c) removing the organic solvent from the separated polysaccharide to obtain a polysaccharide.

In step (a), if the reaction temperature is too low, the extract is obtained in only a small amount and the process does not efficiently proceed. On the contrary, if the reaction temperature is too high, it is possible to degenerate some polysaccharides. In addition, if the reaction time is too short, the extract is obtained in only a small amount. On the contrary, if the reaction time is too long, it is not preferably in terms of economic efficiency. The term "cold-infusing" means immersion in water at approximately 0-25°C, and the term "heating" means immersion in water at approximately 50-180°C. The amount of water to be used is not particularly limited. For better extraction, water is used in an 8-20-fold volume of the white Panax ginseng roots. In the specific Example of the present invention, 1 kg of 6 year-old white Panax ginseng roots was pulverized and dried, followed by extraction using 8 L of water.

In step (b), for effective recovery of the polysaccharides, the liquid extract is concentrated, for example, under reduced pressure of vacuum - 760 mmHg and by heating at 20-90°C. An organic solvent is added to the concentrated liquid extract to precipitate polysaccharides, and the preferred organic solvent is 0.1-1 M sodium chloride and 0.1-1 M lower alcohol or acetone. The organic solvent is used in a 1-5-fold, preferably 2-4-fold volume of the liquid extract. Examples of the lower alcohol may include C1-C5 alcohols such as methanol, ethanol, propanol, butanol and pentanol, preferably ethanol, and more preferably 70-90% (v/v) ethanol. The polysaccharide separation may be performed by the various methods such as filtration (including Ultrafiltration), centrifugation, ion exchange resin, adsorption resin, and dialysis. In the specific Example of the present invention, the liquid extract was distilled under reduced pressure at 56°C, and then 80% (v/v) ethanol was added to the concentrated Panax ginseng solution to obtain a precipitate, followed by dialysis with water for separation.

In step (c), after removing the organic solvent, the resultant may be dried to obtain the polysaccharide in powder form. The drying process may be performed by hot air drying, vacuum drying, freeze-drying, spray drying or the like. In the specific Example of the present invention, the liquid separated in step (b) was freeze-dried to prepare the white Panax ginseng polysaccharide.

Although the method for extracting the white Panax ginseng polysaccharide according to the present invention has been described, those skilled in the art will appreciate that various modifications or variations of the present invention are possible in order to increase extraction efficiency or purity of the white Panax ginseng polysaccharide. Such modifications and variations are intended to fall within the scope of the present invention.

In still another embodiment, the present invention relates to a composition for use in the prevention and treatment of liver diseases, comprising the white Panax ginseng polysaccharide extract as an active ingredient, and a composition for use in inhibiting hepatic fibrosis, comprising the white Panax ginseng polysaccharide extract as an active ingredient.

When the white Panax ginseng polysaccharide of the present invention is used as a medicine, a variety of known methods can be employed in the preparation of pharmaceutical formulations and administration method thereof. When the Panax ginseng polysaccharide is formulated into a pharmaceutical composition for liver protection or for the prevention and treatment of liver diseases, a variety of auxiliaries that are generally used in the preparation of medicines, such as carriers and other additives, for example, stabilizers, emollients, or emulsifiers, may be used, as long as they do not cause adverse effects on the active ingredient. In addition to such active ingredients, the composition of the present invention may contain pharmaceutically suitable, physiologically acceptable adjuvants, and examples of the adjuvants may include solubilizer such as solvents, disintegrants, sweeteners, binders, coating agents, blowing agents, lubricants, glidants, and flavors.

For administration, the pharmaceutical composition of the present invention may also contain at least one pharmaceutically acceptable carrier, in addition to the active ingredients as described above.

Pharmaceutically acceptable carriers may be saline solution, sterile water, Ringer's solution, buffered saline, dextrose solution, maltodextrin solution, glycerol, ethanol or a mixture of one or more thereof. If necessary, other common additives such as antioxidants, buffered solutions, and bacteriostatic agents may be used together. Moreover, the composition may additionally include diluents, dispersants, surfactants, binders, and lubricants in order to formulate it into injectable formulations such as aqueous solution, suspension, and emulsion, or pills, capsules, granules, and tablets. Furthermore, the composition may preferably be formulated depending on particular diseases and its components, using the method described in Remington's Pharmaceutical Science (latest edition), Mack Publishing Company, Easton PA, which is a suitable method in the relevant field of art.
The composition used in the present invention may be prepared in any form such as granule, powder, coated tablet, tablet, capsule, suppository, syrup, juice, suspension, emulsion, drop or injectable liquid formulation, and sustained release formulation of the active ingredient(s).
The content of the Panax ginseng polysaccharide composition used in the present invention may vary widely depending on the type of pharmaceutical formulation, in accordance with the typical methods.
The composition used in the present invention may be administered via typical routes, such as intravenous, intraarterial, intraperitoneal, intramuscular, intrasternal, transdermal, intranasal, inhalational, local, rectal, oral, intraocular, and intradermal routes, depending on the purpose.
The dosage of Panax ginseng polysaccharide that is an active ingredient of the pharmaceutical composition of the present invention may vary depending on patient's condition and severity of liver disease, and it may be administered at a daily dosage of about 10 mg/kg ∼ 500 mg/kg for adults. The amount of the composition can vary depending on various factors, including the kind and severity of diseases, the kind and content of an active ingredient and other components contained in the composition, the kind of a formulation, and patient's age, weight, general health condition, sex and diet, and administration time, administration route, the secretion % of the composition, administration period, and the kind of drugs used in combination.

The present disclosure also relates to a food composition having effects of liver protection and improving liver function, comprising the Panax ginseng polysaccharide extract as an active ingredient.
The food composition of the present disclosure includes all kinds of food products such as functional foods, nutritional supplements, nutrients, pharmafoods, health foods, nutraceuticals, designer foods, and food additives. Examples of the foods that can be prepared by adding the Panax ginseng polysaccharide thereto include all kinds of foods, drinks, gums, tea, vitamin complex, and functional foods.
Further, the Panax ginseng polysaccharide used in the present invention may be added to foods or drinks for the purpose of protecting liver or preventing liver diseases. In this regard, the extract may be added to the food or drink in an amount of 0.01 to 20% by weight, based on the total weight of the food, and also added in an amount of 0.02∼10 g, based on 100 ml of the health drink composition.
The health drink composition of the present disclosure may include, without limitation, other typical additives such as savoring agents, nutrients, vitamin, mineral (electrolyte), flavoring agents (synthetic and natural flavoring agent), colorant, filler (cheese, chocolate, etc), pectic acid and its salts, alginic acid and its salts, organic acid, protective colloidal thickener, pH modifier, stabilizer, preservative, glycerin, alcohol, carbonating agent used in carbonated beverages, except that it contains the Panax ginseng polysaccharide composition as an essential ingredient at an indicated ratio. As the flavoring agents other than those mentioned above, natural flavoring agents (thaumatine, stevia extract (e.g. rebaudioside A), glycyrrhizin), and synthetic flavoring agents (saccharin, aspartame) can be used with advantage. In addition, the compositions used in the present invention may include pulp for the preparation of natural fruit juice, fruit juice drink, and vegetable drink. Such ingredients may be used singly or in any combination thereof. The proportion of the additives is generally in the range of about 0 to 20 parts by weight, per 100 parts by weight of the composition of the present invention.

### Mode for the Invention

Hereinafter, the present invention will be described in detail with reference to the following Preparation Examples and Experimental Examples. However, these Preparation Examples and Experimental Examples are for illustrative purposes only, and the invention is not intended to be limited by these Preparation Examples and Experimental Examples.

### Preparation Example 1. Preparation of Panax ginseng polysaccharide

1 kg of 6 year-old Panax ginseng was pulverized and dried, and then extracted with 8 L of water. The water-soluble fraction was distilled under reduced pressure at 56°C, and the concentrated Panax ginseng solution was saturated with 80 (v/v)% ethanol to obtain a precipitate. Then, the precipitate was subjected to dialysis with water, and then freeze-dried to prepare a Panax ginseng polysaccharide.

### Preparation Example 2. Sugar composition analysis of Panax ginseng polysaccharide

2 ml of 2M-HCl was added to 20 mg of the Panax ginseng polysaccharide of Preparation Example 1, and heated at 100°C for 24 hrs for acid hydrolysis, followed by centrifugation at 1200 rpm for 5 min. The supernatant was taken, 2 M-NaOH was added thereto for neutralization, and concentrated under reduced pressure. Then, 400 *µ*ℓ of 0.3 M-NaOH was added thereto. 100 *µ*ℓ of monosaccharide standard 100 nM/ 0.3.M-NaOH was prepared, and 100 *µ*ℓ of 0.5 M-PMP (1-phenyl-3-methyl-5-pyrazolone) methanol solution was added to each 100 *µ*ℓ of the monosaccharide standard solution and a sample, and reacted with each other at 70°C for 30 min. Then, the solution was cooled at room temperature, and neutralized with 100 *µ*ℓ of 0.3 M-HCl. The residual PMP was removed by chloroform extractions. The supernatant aqueous layer was concentrated, and diluted with 2-fold water, passed through a 0.45 *µ*m-pore size filter. High performance liquid chromatography was performed under the following conditions.
Apparatus: Waters HPLC System (515 HPLC pumps)
Detector: Waters 490E UV Detector (detection wavelength: 250 nm)
Column: Zorbax Extended-C18 column (4.6 cm I.d x 250 mm, 5 *µ*m)
Column temperature: 35°C
Flow rate: 1 ml/min
Sample injection amount: 10 *µ*ℓ
Mobile phase: 50 mM Sodium phosphate (pH 6.9)
   - Buffer A: 15% Acetonitrile (ACN) in Sod. phosphate buffer,
   - Buffer B: 40% Acetonitrile (ACN) in Sod. phosphate buffer
[Gradient conditions] time (min) %A %B
   Initial 100 0
   10 92 8
   30 80 20
   50 100 0

Table 1

**[Table 1]**

| [Table] | | |
|---|---|---|
| Monos accharide | Retention time (RT) | Composition (% by weight) |
| D(+)-mannose | 14.50 | 1.2-2.8 |
| L(+)-rhamnose | 18.82 | Not detected |
| D-glucuronic acid | 19.68 | Not detected |
| D(+)-galacturonic acid | 21.75 | Not detected |
| D(+)-glucose | 25.15 | 4.1-82.8 |
| D(+)-galactose | 27.06 | 10.0-26.6 |
| D(+)-xylose | 28.28 | Not detected |
| L(+)-arabinose | 28.72 | 0.6-5.4 |
| D(+)-fucose | 31.38 | Not detected |

As shown in Table 1, the Panax ginseng polysaccharide is composed of sugars including mannose of 1.2∼2.8% by weight, glucose of 64.1∼82.8% by weight, galactose of 10.0∼26.6% by weight, and arabinose of 0.6∼5.4% by weight.

### (Experimental Example)

ThePanax ginseng polysaccharide was prepared by the above methods of Preparation Examples, and in vivo and in vitro experiments were performed on carbon tetrachloride-induced hepatotoxicity in animals.

### Experimental Example 1. Hepatotoxicity study of single administration of Panax ginseng polysaccharide

Male BALB/c mice were treated by intraperitoneal injection with Panax ginseng polysaccharide at a dose of 10, 50, 100, and 200 mg/kg. After 48 hrs, blood was collected from the mice, and blood plasma was separated. Then, the levels of serum biomarkers of hepatic injury including ALT, AST, ALP, total bilirubin, and albumin were measured. A control group was treated with a phosphate buffer solution in the same manner. Regardless of concentration, no significant changes in the levels of serum biomarkers of hepatic injury were observed in the single administration group, as compared to the control group (see FIG. 1).

### Experimental Example 2. Effects of long-term administration of Panax ginseng polysaccharide on liver

Male BALB/c mice (four mice per group) were orally administrated with Panax ginseng polysaccharide at a dose of 100 mg/kg twice a week for 20 weeks. Every two weeks, blood was taken from the eyes of four mice per group, and sera were separated. The serum biomarker, ALT levels were assessed in order to confirm whether the long-term administration induces hepatotoxicity. Even though total 4000 mg/kg of Panax ginseng polysaccharide was orally administered for 20 weeks, changes in ALT and AST levels were not observed, as compared to the control group, indicating no hepatotoxic action of long-term administration of Panax ginseng polysaccharide (see Table 2).

Table 2

**[Table 2]**

| [Table] | | | | | |
|---|---|---|---|---|---|
| Effects of Panax ginseng polysaccharide on serum biomarkers of hepatic injury after oral administration for 20 weeks | | | | | |
| week | Cumulative dose of Panax ginseng polysaccharide( mg/kg) | Control group | | Panax ginseng administration group | |
| | | ALT(UI/L) | AST(UI/L) | ALT(UI/L) | AST(UI/L) |
| 2 | 400 | 59 | 106 | 52 | 105 |
| 4 | 800 | 60 | 101 | 46 | 98 |
| 6 | 1200 | 50 | 116 | 46 | 104 |
| 8 | 1600 | 59 | 116 | 50 | 107 |
| 10 | 2000 | 52 | 116 | 49 | 105 |
| 12 | 2400 | 54 | 111 | 49 | 105 |
| 14 | 2800 | 60 | 103 | 50 | 100 |
| 16 | 3200 | 55 | 113 | 49 | 97 |
| 18 | 3600 | 59 | 107 | 53 | 99 |
| 20 | 4000 | 55 | 116 | 49 | 109 |

### Experimental Example 3. Hepatoprotective effect of Panax ginseng polysaccharide on carbon tetrachloride-induced liver injury

In order to evaluate hepatoprotective effect of Panax ginseng polysaccharide, the levels of serum biomarkers of hepatic injury were measured in carbon tetrachloride-induced liver injury mouse. To male BALB/c mice, Panax ginseng polysaccharide was administered by intraperitoneal injection at a dose of 100 mg/kg, and silymarin, widely used for the treatment of hepatic disorders, was also administered by intraperitoneal injection at a dose of 400 mg/kg as a positive control. After 24 hrs, carbon tetrachloride (15 *µ*g/kg) was dissolved in oil, and administered by intraperitoneal injection. After 24 hrs, blood was collected from the mice, and blood plasma was separated. Then, the ALT and AST levels were measured. As a control group, phosphate buffer and olive oil were administered in the same manner.

As a result, each of the ALT and AST levels was reduced by 68% and 60% **in the sillmarin-treated liver injury groupe, whereas each of the ALT and AST levels was reduced by 82% and 87% in the Panax ginseng polysaccharide-treated liver injury group, as compared to the carbon tetrachloride-induced liver injury group, indicating excellent hepatoprotective effect of Panax ginseng polysaccharide (see** **FIG. 2****).**

### Experimental Example 4. Hepatoprotective effect of Panax ginseng polysaccharide on acetaminophen-induced liver injury

Acetaminophen, also known as Tylenol, was reported to cause serious **liver injury when it was taken excessively or with alcohol. In orde**r **to evaluate hepatoprotective effect of Panax ginseng polysaccharide on acetaminophen-induced liver injury, Panax ginseng polysaccharide was administered to male BALB/c mice by intraperitoneal injection at a dose of 100 mg/kg. After 24 hrs, acetaminophen (500 mg/kg) was dissolved in warm phosphate buffer, and administered by in-traperitoneal injection. After 24 hrs, blood was collected from the mice, and blood plasma was separated. Then, the ALT and AST levels were measured. As a control group, phosphate buffer was administered in the same manner.**

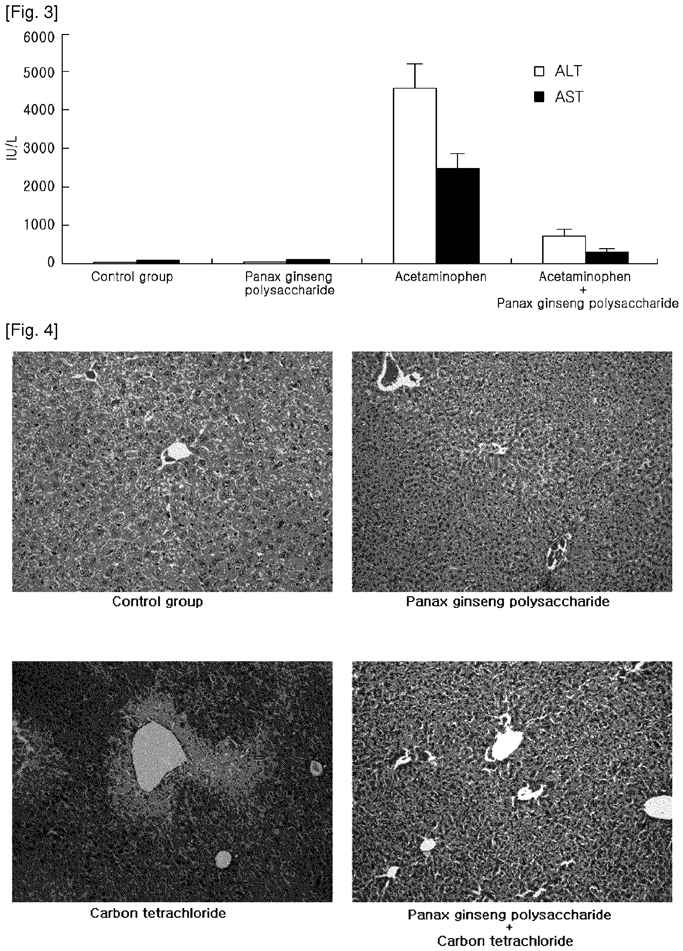

**As a result, each of the ALT and AST levels was reduced by 84% and 87% in the Panax ginseng polysaccharide-treated liver injury group, as compared to the acetaminophen-induced liver injury group, indicating excellent hepatoprotective effect of Panax ginseng polysaccharide on acetaminophen-induced liver injury as well as carbon tetrachloride-induced liver injury (see** **FIG. 3****).**

### Experimental Example 5. Effects of Panax ginseng polysaccharide on lipid per-oxidation in liver

Influx of drug into the liver increases its metabolism and oxidation to **generate lipid peroxide, which is a cause of increasing liver injury. The Panax ginseng polysaccharide-treated liver injury group showed excellent hepatoprotective effect, as compared to the carbon tetrachloride-induced liver injury control group, and thus their liver tissues were collected in order to measure lipid peroxide. The liver tissues were pulverized with saline solution, and then the levels of malonyldialdehyde (MDA), a product of lipid peroxidation, were measured by ThioBarbituric Acid Reactive Substances assay (TBARS assay kit).**

**As a result, the MDA level was significantly increased in the carbon tetra-chloride-induced liver injury group, whereas the administration of Panax ginseng polysaccharide decreased the MDA level by 43%, indicating inhibitory effects of Panax ginseng polysaccharide on carbon tetrachloride-induced lipid peroxidation.**

Table 3

**[Table 3]**

| [Table] | | | | |
|---|---|---|---|---|
| Measurement of lipid peroxide after administration of Panax ginseng polysaccharide and carbon tetrachloride by intraperitoneal injection | | | | |
| MDA measured value | Control group | Panax ginseng polysaccharide | Carbon tetrachloride | Panax ginseng polysaccharide+ Carbon tetrachloride |
| nmole/ml | 10.98±0.83 | 0.96±1.41 | 22.16±1.02 | 12.63±0.67 |

### Experimental Example 6. Immunohistochemical study of liver tissue

In order to examine the hepatoprotective effect of Panax ginseng polysaccharide on carbon tetrachloride-induced liver injury by anatomical/pathological study, Panax ginseng polysaccharide (100 mg/kg) or phosphate buffer was administered to male BALB/c mice by intraperitoneal injection. After 24 hrs, carbon tetrachloride (15 *µ*g/kg) was dissolved in olive oil, and administered by intraperitoneal injection. After 24 hrs, the liver was excised, fixed with paraformalin, and embedded in paraffin. Then, the tissue was cut into slices about 3-5 *µ*m thick, and stained with hematoxylin-eosin to prepare tissue slices, which were examined under a microscope.

As a result, no pathological change was observed in Panax ginseng polysaccharide-treated group, whereas a wide range of necrosis was observed in the carbon tetrachloride-induced liver injury group, as compared to the control group (see FIG. 4).

### Experimental Example 7. Effects of Panax ginseng pollsaccharide on cytochrome P450 and inflammatory cytokine production

### 7-1. Measurement of cytochrome P450-2E1 protein level

A number of drugs are metabolized by the liver through the cytochrome P450 pathway, and carbon tetrachloride is mainly metabolized by cytochrome P450-2E1. Expression of cytochrome P450-2E1 was examined in the liver tissue by Western blotting. In the same manner as described above, Panax ginseng polysaccharide or phosphate buffer was administered. After 24 hrs, carbon tetrachloride was injected to induce liver injury. After 24 hrs, liver tissues were collected, and proteins were extracted therefrom by the typical method. The proteins were separated using a 10% SDS-PAGE gel, and reacted with anti-cytochrome P450-2E1 antibody to examine its expression.

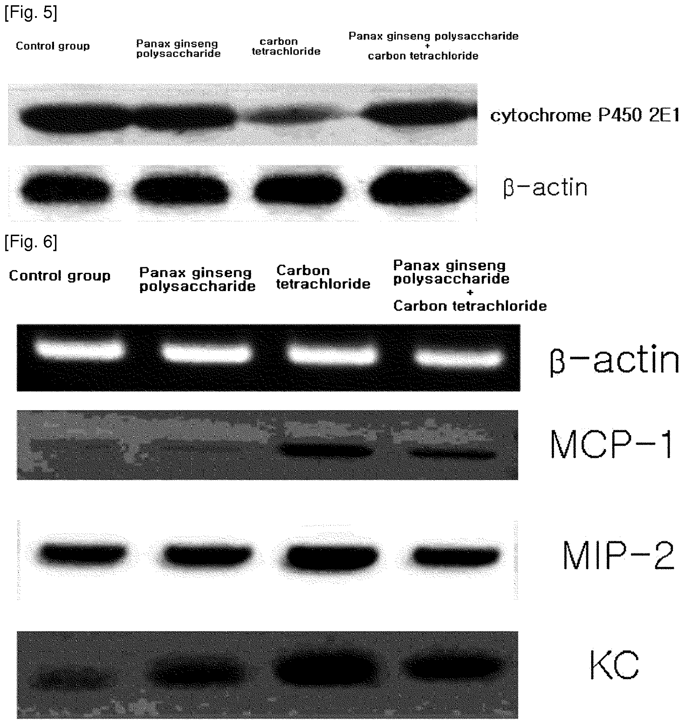

As a result, the expression of cytochrome P450-2E1 was significantly reduced in the carbon tetrachloride-treated group. In contrast, no difference in cytochrome P450-2E1 expression was observed between the control group and the Panax ginseng polysaccharide-treated group. It was also found that the reduced cytochrome P450-2E1 expression by carbon tetrachloride was restored by Panax ginseng polysaccharide to the normal level (see FIG. 5).

### 7-2. Measurement of inflammatory cytokine expression

It was suggested that some cytokines have been implicated in the development of liver injury. Thus, RNA was isolated from liver tissue to synthesize cDNA, and Reverse transcription polymerase chain reaction (RT-PCR) was performed using each cytokine primer. The RT-PCR products were electrophoresed on 1.5% agarose gel to determine their expression levels.

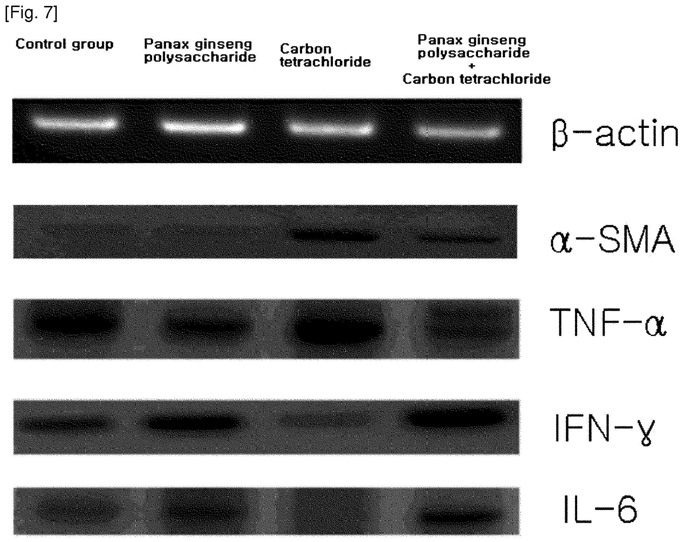

As a result, in the carbon tetrachloride-induced liver injury group, an index of inflammation, TNF-α (Tumor Necrosis Factor-α) expression was increased, whereas the expression of IFN-γ (interferon-γ) or IL-6 (interleukin-6) involved in hepatocyte regeneration was reduced. In contrast, the IFN-γ and IL-6 expressions were slightly increased in Panax ginseng polysaccharide-treated group, as compared to the control group. In addition, Panax ginseng polysaccharide reduced the increased TNF-α expression and increased the reduced IFN-γ and IL-6 expressions in carbon tetrachloride-induced liver injury group. Furthermore, an initial index of cell transformation via inflammation or fibrosis, α-SMA (α-Smooth Muscle Actin) was considerably increased in the carbon tetrachloride-induced liver injury group, but significantly reduced by pre-treatment of the Panax ginseng polysaccharide (see FIG. 7).

### 7-3. Measurement of chemokine expression

The dramatic increase in the secretion of chemokines during inflammation results in the selective recruitment of eosinophils or T cells into inflamed tissue. However, the recruitment of immune cells into the liver in response to cell damage may exacerbate the liver injury, and thus chemokine expression was examined. In the same manner as described above, the liver was excised, and RNA was isolated therefrom, followed by RT-PCR. In the carbon tetrachloride-treated group, chemokines including MCP-1 (Monocyte Chemotactic Protein-1), MIP-2 (Macrophage Inflammatory Protein-2), and KC (Keratinocyte-derived chemokine) that recruit eosinophils into inflamed tissue were remarkably increased, but significantly reduced by pre-treatment of the Panax ginseng polysaccharide (see FIG. 6).

### Experimental Example 8. Inhibitory effects of Panax ginseng polysaccharide on hepatic fibrosis

The α-SMA is widely known as a fibrosis index, and its expression levels were examined in the liver tissues of the above experimental groups, and further examined in hepatocytes (HepG2) in vitro, together with other factors. Hepatocytes were treated with a known inducer of fibrosis, TGF-β at a concentration of 5 nM/ml, and then treated with Panax ginseng polysaccharide by varying concentration (12.5-200 *µ*g/mℓ) and time (20 hr before/after), so as to examine the α-SMA expression levels.
As a result, the α-SMA, fibronectin, and collagen levels were considerably reduced, when treated with Panax ginseng polysaccharide (50 *µ*g/ml or higher) at 2 hrs before and after TGF-β treatment, indicating excellent preventive and therapeutic effects of Panax ginseng polysaccharide on hepatic fibrosis (see FIG. 8).

As described in Experimental Examples, it was demonstrated that the white Panax ginseng polysaccharide used in the present invention has an effect of rapid recovery of liver injury that is induced by hepatotoxins such as carbon tetrachloride and acetaminophen, and hepatoprotective effects, thereby showing no hepatotoxicity upon long-term administration. In addition, hepatic fibrosis resulting from severe inflammation or tissue transformation was significantly reduced by pre-treatment or post-treatment of the Panax ginseng polysaccharide, indicating its protective and therapeutic effects on hepatic fibrosis.

## Claims

1. A white Panax ginseng polysaccharide extract for use in the prevention or treatment of liver diseases, comprising mannose of 1.2-2.8% by weight, glucose of 64.1-82.8% by weight, galactose of 10.0-26.6% by weight, and arabinose of 0.6-5.4% by weight, wherein the liver diseases are selected from the group consisting of fatty liver, liver necrosis, hepatic fibrosis, liver cirrhosis and acute/chronic hepatitis.

2. A method for preparing the white Panax ginseng polysaccharide extract according to claim 1 as an ingredient for the prevention or treatment of liver diseases, comprising the steps of:
(a) adding the plant of the genus *Panax ginseng* to water, and then cold-infusing at 0∼25°C or heating it at 50∼180°C for 0.5∼20 hours to obtain a liquid extract;
(b) concentrating the liquid extract by distillation under reduced pressure, and then precipitating and separating the concentrated liquid extract using an organic solvent comprising 70-90% (v/v) ethanol; and
(c) removing the organic solvent from the separated polysaccharide to obtain a polysaccharide.

3. A composition for use in the prevention or treatment of liver diseases, comprising a white Panax ginseng polysaccharide extract comprising mannose of 1.2-2.8% by weight, glucose of 64.1-82.8% by weight, galactose of 10.0-26.6% by weight, and arabinose of 0.6-5.4% by weight as an active ingredient, the composition further comprising pharmaceutically acceptable carriers,
wherein the liver disease are selected from the group consisting of fatty liver, liver necrosis, hepatic fibrosis, liver cirrhosis and acute/chronic hepatitis.

4. A composition for use in inhibiting hepatic fibrosis, comprising a white Panax ginseng polysaccharide extract comprising mannose of 1.2-2.8% by weight, glucose of 64.1-82.8% by weight, galactose of 10.0-26.6% by weight, and arabinose of 0.6-5.4% by weight as an active ingredient, the composition further comprising pharmaceutically acceptable carriers.

## Patentansprüche

1. Polysaccharidextrakt aus weißem *Panax ginseng* zur Verwendung bei der Prävention oder Behandlung von Lebererkrankungen, umfassend 1,2∼2,8 Gewichts-% an Mannose, 64,1∼82,8 Gewichts-% an Glucose, 10,0∼26,6 Gewichts-% an Galactose sowie 0,6∼5,4 Gewichts-% an Arabinose, wobei die Lebererkrankungen ausgewählt sind aus der Gruppe bestehend aus Leberverfettung, Lebernekrose, Leberfibrose, Leberzirrhose und akuter/chronischer Hepatitis.

2. Verfahren zur Herstellung des Polysaccharidextrakts aus weißem *Panax ginseng* nach Anspruch 1 als einen Inhaltsstoff zur Prävention oder Behandlung von Lebererkrankungen, umfassend die folgenden Schritte:
(a) das Beigeben der Pflanze der Gattung *Panax ginseng* zu Wasser sowie das anschließende Kaltaufgießen bei 0∼25°C oder Erhitzen bei 50-180°C für 0,5∼20 h zum Erhalt eines flüssigen Extrakts;
(b) das Aufkonzentrieren des flüssigen Extrakts mittels Destillation unter reduziertem Druck sowie das anschließende Präzipitieren und Separieren des konzentrierten flüssigen Extrakts unter Verwendung eines organischen Lösungsmittels, das 70-90% (v/v) Ethanol umfasst; und
(c) das Entfernen des organischen Lösungsmittels von dem separierten Polysaccharid zum Erhalt eines Polysaccharids.

3. Zusammensetzung zur Verwendung bei der Prävention oder Behandlung von Lebererkrankungen, umfassend einen Polysaccharidextrakt aus weißem *Panax ginseng,* umfassend 1,2∼2,8 Gewichts-% an Mannose, 64,1∼82,8 Gewichts-% an Glucose, 10,0∼26,6 Gewichts-% an Galactose sowie 0,6∼5,4 Gewichts-% an Arabinose als wirksamen Bestandteil, wobei die Zusammensetzung des Weiteren pharmazeutisch verträgliche Träger umfasst,
wobei die Lebererkrankungen ausgewählt sind aus der Gruppe bestehend aus Leberverfettung, Lebernekrose, Leberfibrose, Leberzirrhose und akuter/chronischer Hepatitis.

4. Zusammensetzung zur Verwendung bei der Inhibition von Leberfibrose, umfassend einen Polysaccharidextrakt aus weißem *Panax ginseng,* umfassend 1,2∼2,8 Gewichts-% an Mannose, 64,1∼82,8 Gewichts-% an Glucose, 10,0∼26,6 Gewichts-% an Galactose sowie 0,6∼5,4 Gewichts-% an Arabinose als wirksamen Bestandteil, wobei die Zusammensetzung des Weiteren pharmazeutisch verträgliche Träger umfasst.

## Revendications

1. Extrait de polysaccharide de Panax ginseng blanc pour une utilisation dans la prévention ou le traitement de maladies du foie, comprenant du mannose à 1,2-2,8 % en poids, du glucose à 64,1-82,8 % en poids, du galactose à 10,0-26,6 6 % en poids et de l'arabinose à 0,6-5,4 % en poids, dans lequel les maladies du foie sont choisies dans le groupe consistant en la stéatose hépatique, la nécrose du foie, la fibrose hépatique, la cirrhose du foie et l'hépatite aiguë/chronique.

2. Procédé de préparation de l'extrait de polysaccharide de Panax ginseng blanc selon la revendication 1 comme ingrédient pour la prévention ou le traitement de maladies du foie, comprenant les étapes consistant à :
(a) ajouter la plante du genre *Panax ginseng* à de l'eau, puis infuser à froid à 0∼25°C ou chauffer à 50∼180°C pendant 0,5∼20 heures pour obtenir un extrait liquide ;
(b) concentrer l'extrait liquide par distillation sous pression réduite, puis faire précipiter et séparer l'extrait liquide concentré à l'aide d'un solvant organique comprenant 70-90 % (v/v) d'éthanol ; et
(c) retirer le solvant organique du polysaccharide séparé pour obtenir un polysaccharide.

3. Composition pour une utilisation dans la prévention ou le traitement de maladies du foie, comprenant un extrait de polysaccharide de Panax ginseng blanc comprenant du mannose à 1,2-2,8 % en poids, du glucose à 64,1-82,8 8 % en poids, du galactose à 10,0-26,6 % en poids et de l'arabinose à 0,6-5,4 % en poids comme principe actif, la composition comprenant de plus des supports pharmaceutiquement acceptables,
dans laquelle les maladies du foie sont choisies dans le groupe consistant en la stéatose hépatique, la nécrose du foie, la fibrose hépatique, la cirrhose du foie et l'hépatite aiguë/chronique.

4. Composition pour une utilisation dans l'inhibition de la fibrose hépatique, comprenant un extrait de polysaccharide de Panax ginseng blanc comprenant du mannose à 1,2-2,8 % en poids, du glucose à 64,1-82,8 % en poids, du galactose à 10,0-26,6 % en poids et de l'arabinose à 0,6-5,4 % en poids comme principe actif, la composition comprenant en outre des supports pharmaceutiquement acceptables.
